# EUROPEAN PATENT APPLICATION

(11) **EP 4 722 719 A1**
(43) Date of publication of application: **08.04.2026**
(21) Application number: 24819127.2
(22) Date of filing: 17.05.2024
(51) Int. Cl.: G01N 33/50, G01N 27/62

(54) **BLOOD INSPECTION METHOD**

(30) Priority: 05.06.2023 JP 2023092194
(71) Applicant: SHIMADZU CORPORATION, Kyoto-shi, Kyoto 604-8511 (JP); Jichi Medical University, Tokyo 102-0093 (JP)
(72) Inventor: GODA, Takahiro, Kyoto-shi, Kyoto 604-8511 (JP); AIZAWA, Kenichi, Shimotsuke-shi, Tochigi 329-0498 (JP); KIMURA, Natsuka, Shimotsuke-shi, Tochigi 329-0498 (JP)
(74) Representative: Müller-Boré & Partner Patentanwälte PartG mbB
(86) International application number: PCT/JP2024/018266
(87) International publication number: WO 2024/252894

(57) **Abstract**

A blood testing method comprising:
a preparation step of preparing whole blood drawn from a subject;
a pretreatment step of pretreating the whole blood to prepare an analysis sample;
a separation step of separating the analysis sample into components by using a liquid chromatograph; and
an analysis step of analyzing the separated components by using a mass spectrometry apparatus, wherein
the subject is a subject receiving administration of a first immunosuppressant and a second immunosuppressant,
the first immunosuppressant includes at least one selected from the group consisting of tacrolimus, cyclosporine A, everolimus, and sirolimus,
the second immunosuppressant includes at least one selected from the group consisting of mycophenolic acid and metabolites thereof,
the separated components include the first immunosuppressant and the second immunosuppressant, and
in the analyzing by using a mass spectrometry apparatus, the analyzing is performed in a positive mode when a component to be analyzed is the first immunosuppressant, and the analyzing is performed in a negative mode when a component to be analyzed is the second immunosuppressant.

## Description

### TECHNICAL FIELD

The present invention relates to a blood testing method.

### BACKGROUND ART

In transplantation therapy with organs or hematopoietic stem cells, for the purpose of reducing rejection due to transplantation, immunosuppressants are usually administered to the patients. However, excess administration of immunosuppressants can excessively reduce immunocompetence of the patients and cause infectious diseases and the like. For this reason, it is necessary to measure and/or monitor the drug concentration in blood (also called "the blood drug concentration") after administration of immunosuppressants to closely control the dose.

Known methods for monitoring blood drug concentrations (therapeutic drug monitoring; hereinafter also abbreviated as "TDM") include separation/analysis by high performance liquid chromatography or gas chromatography, immunological techniques such as fluorescence polarization immunoassay, enzyme immunoassay, and radioactive immunoassay, as well as atomic absorption spectrometry.

For instance, Japanese Patent Laying-Open No. 2021-128005 (PTL 1) discloses a method for monitoring blood drug concentrations, which comprises loading a deproteinized blood specimen on a pre-conditioned solid-phase extraction column, letting the blood specimen pass through the solid-phase extraction column, washing the solid-phase extraction column, eluting the adsorbed matter from the solid-phase extraction column with the use of elution liquid, and analyzing the eluate by LC-MS/MS.

### CITATION LIST

### PATENT LITERATURE

PTL 1: Japanese Patent Laying-Open No. 2021-128005

### SUMMARY OF INVENTION

### TECHNICAL PROBLEM

The blood sample prepared for monitoring blood drug concentrations can be whole blood or plasma depending on the monitoring-target drug. For instance, for monitoring immunosuppressants such as tacrolimus, cyclosporine A, everolimus, and sirolimus, whole blood is used as the blood sample. On the other hand, for monitoring other immunosuppressants such as mycophenolic acid and metabolites thereof (for example, mycophenolic acid β-D-glucuronide), plasma is used as the blood sample. Because of this, when it is intended to monitor two or more types of drugs and therefore it is necessary to prepare both whole blood and plasma, a heavy burden is on the patient due to, among others, a great amount of blood required to be drawn.

The present invention has been devised in light of the above-described circumstances, and aims at providing a blood testing method capable of quantitatively assessing two or more types of immunosuppressants in the same blood sample.

### SOLUTION TO PROBLEM

The inventors of the present invention have conducted intensive research, and as a result, have found that it is possible to quantitatively assess two or more types of immunosuppressants in the same blood sample, by using whole blood as the blood sample and changing the ionization mode in mass spectrometry in accordance with the component to analyze. Thus, the present invention has now been completed.

A first aspect of the present invention relates to a blood testing method comprising:
a preparation step of preparing whole blood drawn from a subject;
a pretreatment step of pretreating the whole blood to prepare an analysis sample;
a separation step of separating the analysis sample into components by using a liquid chromatograph; and
an analysis step of analyzing the separated components by using a mass spectrometry apparatus, wherein
the subject is a subject receiving administration of a first immunosuppressant and a second immunosuppressant,
the first immunosuppressant includes at least one selected from the group consisting of tacrolimus, cyclosporine A, everolimus, and sirolimus,
the second immunosuppressant includes at least one selected from the group consisting of mycophenolic acid and metabolites thereof,
the separated components include the first immunosuppressant and the second immunosuppressant, and
in the analyzing by using a mass spectrometry apparatus, the analyzing is performed in a positive mode when a component to be analyzed is the first immunosuppressant, and the analyzing is performed in a negative mode when a component to be analyzed is the second immunosuppressant.

### ADVANTAGEOUS EFFECTS OF INVENTION

The present invention makes it possible to provide a blood testing method capable of quantitatively assessing two or more types of immunosuppressants in the same blood sample.

### DESCRIPTION OF EMBODIMENTS

In the following, a description will be given of an embodiment of the present invention (hereinafter referred to as "the present embodiment"). It should be noted that the present embodiment is not limited to the below description. In the present specification, the expression "(from) A to Z" means the upper limit and the lower limit of a range (that is, it means not less than A and not more than Z), and if a description of unit is attached only to Z but not to A, A has the same unit as that of Z.

### <<Blood Testing Method>>

A first aspect of the present invention relates to a blood testing method comprising:
a preparation step of preparing whole blood drawn from a subject;
a pretreatment step of pretreating the whole blood to prepare an analysis sample;
a separation step of separating the analysis sample into components by using a liquid chromatograph; and
an analysis step of analyzing the separated components by using a mass spectrometry apparatus, wherein
the subject is a subject receiving administration of a first immunosuppressant and a second immunosuppressant,
the first immunosuppressant includes at least one selected from the group consisting of tacrolimus, cyclosporine A, everolimus, and sirolimus,
the second immunosuppressant includes at least one selected from the group consisting of mycophenolic acid and metabolites thereof,
the separated components include the first immunosuppressant and the second immunosuppressant, and
in the analyzing by using a mass spectrometry apparatus, the analyzing is performed in a positive mode when a component to be analyzed is the first immunosuppressant, and the analyzing is performed in a negative mode when a component to be analyzed is the second immunosuppressant.

### <Preparation Step>

This step involves preparing whole blood drawn from a subject. The subject is a subject receiving administration of a first immunosuppressant and a second immunosuppressant. "Subject" means a human from which the whole blood for the blood testing method according to the present embodiment is drawn, and the age, sex, and race are not particularly limited. The subject may be a healthy subject, or may be a patient having a disease of some kind. In one aspect of the present embodiment, the subject is preferably a patient who is receiving transplantation therapy with an organ or hematopoietic stem cells.

In the present embodiment, "whole blood" means blood as it was drawn from the subject, or a blood sample prepared by adding an anticoagulant to the blood. The whole blood contains blood cell components, platelets, and plasma components. The method for drawing the whole blood is not particularly limited, and a known method may be used for drawing whole blood from the subject.

In the present embodiment, the first immunosuppressant includes at least one selected from the group consisting of tacrolimus (the generic name is tacrolimus hydrate), cyclosporine A, everolimus, and sirolimus.

In the present embodiment, the second immunosuppressant includes at least one selected from the group consisting of mycophenolic acid (MPA) and metabolites thereof (for example, mycophenolic acid β-D-glucuronide (MPAG)).

### <Pretreatment Step>

This step involves pretreating the whole blood to prepare an analysis sample.

The method of pretreating the whole blood is not particularly limited as long as it enables separation of the pretreated analysis sample into components by a liquid chromatograph described below. In one aspect of the present embodiment, "analysis sample" may be a sample that is derived from the whole blood and is to be subjected to a separation step described below. Examples of the pretreatment include protein removal operation (also referred to as "deproteinization operation"), extraction based on solid-phase extraction, and the like.

In one aspect of the present embodiment, prior to the pretreatment, a certain amount of an internal standard may be added to the whole blood. As the internal standard, a known internal standard can be used, for example.

### <Separation Step>

This step involves separating the analysis sample into components by using a liquid chromatograph. The liquid chromatograph (LC) uses the difference in affinity of the components in the analysis sample for a mobile phase and a stationary phase, to separate the components and elute them at different retention times. The type of the liquid chromatograph is not particularly limited as long as it is capable of separating the components with a desired level of accuracy so that an analysis-target component can be separated and detected on a mass spectrometry apparatus. As the liquid chromatograph, a nano LC, a micro LC, a high performance liquid chromatograph (HPLC), an ultra high performance liquid chromatograph (UHPLC), and the like can be used, for example.

In the present embodiment, the mobile phase is not particularly limited; for example, as a mobile phase A, an ammonium formate aqueous solution may be used, and as a mobile phase B, a liquid containing acetonitrile and isopropanol in a certain proportion such as a volume ratio of 1:1, as well as a mobile phase described below in Examples may be used.

In the present embodiment, the stationary phase is not particularly limited; examples thereof include silane supported on a support such as silica gel and having a linear hydrocarbon such as C8 and/or C18 bonded thereto, as well as a stationary phase described below in Examples.

### <Analysis Step>

This step involves analyzing the separated components by using a mass spectrometry apparatus. The separated components include the first immunosuppressant and the second immunosuppressant.

In the present embodiment, in the analyzing by using the mass spectrometry apparatus, the analyzing is performed in a positive mode when the analysis-target component is the first immunosuppressant, and the analyzing is performed in a negative mode when the analysis-target component is the second immunosuppressant. In one aspect of the present embodiment, when the elution time of the target component in the liquid chromatograph separation is known in advance, it is possible to set the ionization mode to either a positive mode or a negative mode based on the elution time.

Conventionally, for monitoring immunosuppressants such as tacrolimus, cyclosporine A, everolimus, and sirolimus (first immunosuppressants), whole blood is used as the blood sample. On the other hand, for monitoring other immunosuppressants such as mycophenolic acid and metabolites thereof (for example, mycophenolic acid β-D-glucuronide) (second immunosuppressants), plasma is used as the blood sample. Because of this, when it is intended to monitor both the first immunosuppressant and the second immunosuppressant and therefore it is necessary to prepare both whole blood and plasma, a heavy burden is on the patient due to, among others, a great amount of blood required to be drawn. Another reason for the great amount of blood required to be drawn is that the concentration range at which the first immunosuppressant is detectable is greatly different from the concentration range at which the second immunosuppressant is detectable. At the time when measuring them with LC-MS, the blood concentration of the second immunosuppressant is higher than the blood concentration of the first immunosuppressant. Because of this, when sensitivity is optimized for detection of the first immunosuppressant, for instance, saturation occurs for detection of the second immunosuppressant; hence, simultaneous analysis of both components is not easy to perform.

The inventors of the present invention have conducted intensive research, and as a result, have found that it is possible to quantitatively assess two or more types of immunosuppressants (the first immunosuppressant and the second immunosuppressant) in the same blood sample, by using whole blood as the blood sample and changing the ionization mode in mass spectrometry in accordance with the component to analyze. Conventionally, for lowering the sensitivity of mass spectrometry, a negative mode is used and/or the gas flow rate or the voltage is changed. However, use of a negative mode at the time of analysis of the second immunosuppressant in order to simultaneously analyze the first immunosuppressant and the second immunosuppressant in the same blood sample, namely to unify the dynamic range, has never been performed.

The method of ionization with the mass spectrometry apparatus is not particularly limited, and electrospray (ESI), nanoelectrospray ionization (nano-LSI), and the like can be used. In the present embodiment, the ionization method is preferably ESI.

Examples of the mass spectrometry apparatus include a Fourier transform mass spectrometer, a time-of-flight (Q-TOF) mass spectrometry apparatus, a triple quadrupole mass spectrometry apparatus, and the like. In the present embodiment, from the viewpoint of performing analysis of a known component in a short time with accuracy, the mass spectrometry apparatus is preferably a triple quadrupole mass spectrometry apparatus.

In one aspect of the present embodiment, the separation step and the analysis step may be implemented successively. For instance, a liquid chromatograph-tandem mass spectrometry apparatus may be used for successively implementing the separation step and the analysis step. Typically, the liquid chromatograph-tandem mass spectrometry apparatus is a liquid chromatograph-triple quadrupole mass spectrometry apparatus or a liquid chromatograph-triple quadrupole/time-of-flight (Q-TOF) mass spectrometry apparatus. Usually, for a liquid chromatograph-tandem mass spectrometry apparatus of this type, analysis parameters are determined so as to ensure the highest detection sensitivity possible, namely so as to ensure the best condition, for each component in the eluate separated with the liquid chromatograph.

In one aspect of the present embodiment, it is preferable that the analysis step further include quantitatively assessing the separated components. The method for quantitatively assessing the separated components is not particularly limited, and a known method can be used. Examples of such a method include an absolute calibration technique, an internal standard technique, and the like. In another aspect of the present embodiment, it is preferable that the analysis step further include quantitatively assessing the separated components based on a calibration curve created in advance.

### [Examples]

In the following, a more detailed description will be given of the present invention by way of examples, but these examples are not intended to limit the scope of the present invention.

### <<Creation of Calibration Curve>>

### <Preparation of Whole Blood Calibration Solution>

In the manner described below, a whole blood calibration solution (L1 to L6) was prepared. Firstly, an MPA methanol solution (concentration, 0.01 to 5 mg/L) and an MPAG methanol solution (concentration, 0.1 to 25 mg/L) were added into a microtube, each in 30 µl. Subsequently, the solvent methanol was evaporated so that only MPA and MPAG remained in the microtube. In this way, six microtubes (L1 to L6) containing different amounts of MPA and MPAG were prepared.

To the microtube L1, 3 µl of a calibrator base liquid L1 was added. Herein, "calibrator base liquid" refers to a liquid sample made of whole blood as a matrix in which tacrolimus, cyclosporine A, everolimus, and sirolimus are dissolved in the concentrations specified in Table 1.

Then, to the microtube L1, an internal standard sample (3 µl) included in an immunosuppressant analysis kit, DOSIMMUNE (manufactured by Shimadzu Corporation), an internal standard sample (3 µl) included in an immunosuppressant analysis kit, DOSIMYCO (manufactured by Shimadzu Corporation), and an extraction liquid (a mixed liquid, acetonitrile:methanol:water=40:40:20) (200 µl) were added, and the resultant was mixed with the use of a vortex mixer for 1 minute. After mixed, the microtube L1 was centrifuged (15000 rpm, 7 minutes) so that the supernatant was separated from the precipitate (a pretreatment step). 200 µl of the supernatant was to be used as a whole blood calibration solution L1.

The microtubes L2 to L6 were subjected to the same procedure as the above-described procedure except that 3 µl of calibrator base liquids L2 to L6 were added, respectively, and thereby, whole blood calibration solutions L2 to L6 were prepared. Table 1 shows the concentrations of the components contained in the calibrator base liquids L1 to L6.

**[Table 1]**

| | Component | L1 | L2 | L3 | L4 | L5 | L6 |
|---|---|---|---|---|---|---|---|
| | Cyclosporin A | 26.07 | 106.48 | 453.67 | 1027.00 | 1574.30 | 1866.00 |
| | Sirolimus | 1.93 | 5.07 | 9.76 | 15.09 | 24.30 | 35.40 |
| Calibrator base liquid (µg/L) | Everolimus | 2.04 | 4.88 | 9.20 | 14.77 | 24.84 | 36.88 |
| | Tacrolimus | 1.73 | 4.86 | 9.27 | 13.96 | 23.30 | 33.09 |
| | MPA | 100 | 500 | 5000 | 10000 | 25000 | 50000 |
| | MPAG | 1000 | 5000 | 25000 | 50000 | 125000 | 250000 |

### <Separation and Analysis>

The whole blood calibration solutions L1 to L6 were analyzed by gradient elution on LC-MS/MS. The analysis conditions are as described below.

### (Chromatography Conditions)

Trap solvent (Pump C): DOSIMMUNE MOBILE PHASE A
Mobile phase A (Pump A): DOSIMMUNE MOBILE PHASE A
Mobile phase B (Pump B): DOSIMMUNE MOBILE PHASE B
Initial flow rate of mobile phase: 0.2 mL/min
Trap column: DOSIMMUNE Trap Column (S/N ALIMTCL000000155)
Analytical column: DOSIMMUNE Analytical Column (S/N ALIMTCL000000155)
Initial valve position: 0 (Pump C - Trap column - Drain)
Time program: See Table 2 (The unit of time is "minutes")
Column temperature: 65°C
Amount injected: 20 µL

**[Table 2]**

| Time | Unit | Operation command | Value |
|---|---|---|---|
| 0.01 | Pump | T.Flow | 0.8 |
| 0.01 | Pump | C.Flow | 2 |
| 0.25 | Column oven | Oven Valve 1 | 1 |
| 0.25 | Pump | C.Flow | 2 |
| 0.26 | Pump | C.Flow | 0.02 |
| 1 | Pump | T.Flow | 0.8 |
| 1 | Pump | B.Conc | 60 |
| 1.25 | Pump | T.Flow | 0.8 |
| 1.25 | Pump | B.Conc | 100 |
| 1.5 | Column oven | Oven Valve 1 | 0 |
| 1.5 | Pump | C.Flow | 0.02 |
| 1.51 | Pump | C.Flow | 2 |
| 2.5 | Pump | B.Conc | 100 |
| 2.51 | Pump | B.Conc | 60 |
| 3 | Controller | Stop | |

| | | | |
|---|---|---|---|
| At Oven Valve 1:1, flow is switched; Pump A - Trap column - Analytical column - MS. | | | |

### (Mass Spectrometry Conditions)

Ionization method:
ESI-Negative MRM mode (0-1.25 min for MPA and MPAG)
ESI-Positive MRM mode (1.25-3.00 min for everolimus, sirolimus, tacrolimus, and cyclosporine A)

Nebulizer gas flow rate: 3 L/min
Heating gas flow rate: 10 L/min
Interface temperature: 200°C
Drying gas flow rate: 10 L/min
DL temperature: 150°C
Heat block temperature: 200°C
CID gas pressure: 230 kPa
Probe position: +3 mm

### (MRM Conditions)

MPA (ESI-Negative): 319.2000>191.2500 (CE 30 V)
MPA IS (ESI-Negative): 323.2000>191.2500 (CE 23 V)
MPAG (ESI-Negative): 495.2000>191.3000 (CE 37 V)
MPAG IS (ESI-Negative): 499.2000>191.2000 (CE 42 V)
Everolimus (ESI-Positive): 975.6000>908.5500 (CE -17 V)
Everolimus IS (ESI-Positive): 981.6000>914.7000 (CE -17 V)
Sirolimus (ESI-Positive): 931.6000>864.6500 (CE -17 V)
Sirolimus IS (ESI-Positive): 935.6000>864.5000 (CE -18 V)
Tacrolimus (ESI-Positive): 821.5000>768.5500 (CE -22 V)
Tacrolimus IS (ESI-Positive): 826.5000>773.6000 (CE -22 V)
Cyclosporine A (ESI-Positive): 1220.0000>1202.9500 (CE -19 V)
Cyclosporine A IS (ESI-Positive): 1232.0000>1215.0000 (CE -19 V)

Calibration curves for the components created based on the analysis results are given in Table 3 to Table 8. In Table 3 to Table 8, "Set concentration" is the concentration in the section "Calibrator base liquid" in Table 1, and "Concentration" is the concentration calculated from "Area ratio" based on the calibration curve. "Accuracy %" represents the proportion (%) of the concentration calculated from "Area ratio", relative to "Set concentration".

**[Table 3]**

| MPA calibration curve | | | | | |
|---|---|---|---|---|---|
| | Set concentration (mg/L) | Retention time (min) | Area ratio | Concentration (mg/L) | Accuracy (%) |
| L1 | 0.1 | 0.89 | 0.007 | 0.11 | 106.3 |
| L2 | 0.5 | 0.90 | 0.040 | 0.50 | 100.7 |
| L3 | 5 | 0.90 | 0.399 | 4.90 | 98.1 |
| L4 | 10 | 0.90 | 0.768 | 9.43 | 94.3 |
| L5 | 25 | 0.90 | 2.011 | 24.65 | 98.6 |
| L6 | 50 | 0.90 | 4.162 | 51.01 | 102 |
| Approximate expression, etc. | Y=0.0816333X-0.00152902 | | | | |
| | R² (proportion of variance)=0.9993200, R=0.9996599 | | | | |

**[Table 4]**

| MPAG calibration curve | | | | | |
|---|---|---|---|---|---|
| LEVEL | Set concentration (mg/L) | Retention time (min) | Area ratio | Concentration (mg/L) | Accuracy (%) |
| L1 | 1 | 0.79 | 0.003 | 1.07 | 107.4 |
| L2 | 5 | 0.79 | 0.018 | 5.54 | 110.7 |
| L3 | 25 | 0.78 | 0.073 | 22.07 | 88.3 |
| L4 | 50 | 0.78 | 0.146 | 44.07 | 88.1 |
| L5 | 125 | 0.78 | 0.434 | 130.42 | 104.3 |
| L6 | 250 | 0.78 | 0.842 | 252.84 | 101.1 |
| Approximate expression, etc. | Y=0.00333082X-0.000433601 | | | | |
| | R² (proportion of variance)=0.9967909, R=0.9983942 | | | | |

**[Table 5]**

| Everolimus calibration curve | | | | | |
|---|---|---|---|---|---|
| LEVEL | Set concentration(µg/L) | Retention time (min) | Area ratio | Concentration (µg/L) | Accuracy (%) |
| L1 | 2.04 | 1.67 | 0.064 | 1.8 | 87.8 |
| L2 | 4.88 | 1.67 | 0.137 | 4.5 | 92.9 |
| L3 | 9.20 | 1.67 | 0.290 | 10.2 | 111.4 |
| L4 | 14.77 | 1.67 | 0.475 | 17.1 | 116.1 |
| L5 | 24.84 | 1.67 | 0.671 | 24.5 | 98.5 |
| L6 | 36.88 | 1.67 | 0.937 | 34.4 | 93.3 |
| Approximate expression, etc. | Y=0.0267463X+0.0161442 | | | | |
| | R² (proportion of variance)=0.9868035, R=0.9933798 | | | | |

**[Table 6]**

| Sirolimus calibration curve | | | | | |
|---|---|---|---|---|---|
| LEVEL | Set concentration(µg/L) | Retention time (min) | Area ratio | Concentration (µg/L) | Accuracy (%) |
| L1 | 1.93 | 1.67 | 0.058 | | 99.6 |
| L2 | 5.07 | 1.67 | 0.122 | 4.58 | 90.3 |
| L3 | 9.76 | 1.67 | 0.251 | 9.99 | 102.3 |
| L4 | 15.09 | 1.67 | 0.400 | 16.21 | 107.4 |
| L5 | 24.30 | 1.67 | 0.644 | 26.40 | 108.7 |
| L6 | 35.40 | 1.67 | 0.788 | 32.45 | 91.7 |
| Approximate expression, etc. | Y=0.0239061X+0.0122909 | | | | |
| | R² (proportion of variance)=0.9896347, R=0.99-48039 | | | | |

**[Table 7]**

| Tacrolimus calibration curve | | | | | |
|---|---|---|---|---|---|
| LEVEL | Set concentration(µg/L) | Retention time (min) | Area ratio | Concentration (µg/L) | Accuracy (%) |
| L1 | 1.73 | 1.66 | 0.126 | 1.74 | 100.4 |
| L2 | 4.86 | 1.66 | 0.307 | 4.72 | 97.1 |
| L3 | 9.27 | 1.66 | 0.584 | 9.31 | 100.5 |
| L4 | 13.96 | 1.66 | 0.904 | 14.61 | 104.7 |
| L5 | 23.30 | 1.66 | 1.385 | 22.57 | 96.9 |
| L6 | 33.09 | 1.66 | 2.031 | 33.26 | 100.5 |
| Approximate expression, etc. | Y=0.0604091X+0.0215653 | | | | |
| | R² (proportion of variance)=0.9988657, R=0.9994327 | | | | |

**[Table 8]**

| Cyclosporine A calibration curve | | | | | |
|---|---|---|---|---|---|
| LEVEL | Set concentration(µg/L) | Retention time (min) | Area ratio | Concentration (µg/L) | Accuracy (%) |
| L1 | 26.07 | 1.74 | 0.050 | 23.62 | 90.6 |
| L2 | 106.48 | 1.74 | 0.170 | 102.26 | 96 |
| L3 | 453.67 | 1.74 | 0.769 | 493.75 | 108.8 |
| L4 | 1027.00 | 1.74 | 1.830 | 1186.86 | 115.6 |
| L5 | 1574.30 | 1.74 | 2.317 | 1505.29 | 95.6 |
| L6 | 1866.00 | 1.74 | 2.679 | 1741.74 | 93.3 |
| Approximate expression, etc. | Y=0.00153036X+0.0136225 | | | | |
| | R² (proportion of variance)=0.9908872, R=0.9954332 | | | | |

Referring to the results given in Table 3 to Table 8, it was demonstrated that by performing ionization of everolimus, sirolimus, tacrolimus, and cyclosporine A (four drugs corresponding to the first immunosuppressant) in a positive mode and performing ionization of MPA and MPAG (two drugs corresponding to the second immunosuppressant) in a negative mode, it is possible to create calibration curves of these components from the same analysis sample (whole blood calibration solutions L1 to L6).

### <<Quantitative Assessment of Blood Concentrations of Immunosuppressants>>

### <Preparation of Blood Samples>

As blood samples, the following samples were prepared (a preparation step).
DOSIMMUNE control: manufactured by Shimadzu Corporation, under the trade name "Immunosuppressant analysis kit DOSIMMUNE" (in four concentrations)
MPA MPAG QC whole blood: (in three concentrations)
The DOSIMMUNE control is a sample containing the above-mentioned four drugs corresponding to the first immunosuppressant dissolved in the whole blood matrix. The MPA MPAG QC whole blood is a sample containing the above-mentioned two drugs corresponding to the second immunosuppressant dissolved in the whole blood matrix. Both blood samples are prepared to mimic a blood sample (whole blood) drawn from a subject who received administration of the immunosuppressants.

The MPA MPAG QC whole bloods (L1 to L3) were prepared in the manner described below. Firstly, an MPA methanol solution (concentration, 4000 mg/L) (5 µL) and an MPAG methanol solution (concentration, 2000 mg/L) (50 µL) were added to whole blood (445 µL), and the resultant was mixed with the use of a vortex mixer for 1 minute, to prepare the MPA MPAG QC whole blood (L3). Then, the resulting MPA MPAG QC whole blood (L3) was diluted by a factor of 2 with whole blood, and thereby the MPA MPAG QC whole blood (L2) was obtained. Lastly, the resulting MPA MPAG QC whole blood (L2) was diluted by a factor of 10 with whole blood, and thereby the MPA MPAG QC whole blood (L1) was obtained.

### (Concentrations of Components in MPA MPAG QC Whole Bloods)

L1: MPA 2 mg/L, MPAG 10 mg/L
L2: MPA 20 mg/L, MPAG 100 mg/L
L3: MPA 40 mg/L, MPAG 200 mg/L

### <Pretreatment Step>

The blood sample (3 µl) thus prepared was added to a microtube. Then, to the microtube, an internal standard sample (3 µl) included in an immunosuppressant analysis kit, DOSIMMUNE, an internal standard sample (3 µl) included in an immunosuppressant analysis kit, DOSIMYCO, and an extraction liquid (a mixed liquid, acetonitrile:methanol:water=40:40:20) (200 µl) were added, and the resultant was mixed with the use of a vortex mixer for 1 minute. After mixed, the microtube was centrifuged (15000 rpm, 7 minutes) so that the supernatant was separated from the precipitate (a pretreatment step). 200 µl of the supernatant was to be used as an analysis sample.

### <Separation Step and Analysis Step>

The analysis sample was analyzed by gradient elution on LC-MS/MS. The analysis conditions were the same as those for creating the calibration curves. Results are given in Table 9-1 and Table 9-2.

**[Table 9-1]**

| | CONTROL | | | |
|---|---|---|---|---|
| | Concentration (in specimen) | | | |
| LEVEL | L1 | | L2 | |
| | Quantitatively assessed value | Reference value | Quantitatively assessed value | Reference value |
| MPA (mg/L) | 2.057 | 1.70-2.30 | 20.834 | 17.00-23.00 |
| MPAG (mg/L) | 10.906 | 8.50-11.50 | 90.29 | 85.00-115.00 |
| Everolimus (µg/L) | 3.520 | 3.13-4.70 | 8.287 | 7.34-11.02 |
| Sirolimus (µg/L) | 3.981 | 3.06-4.60 | 7.960 | 6.80-10.20 |
| Tacrolimus (µg/L) | 2.926 | 2.69-4.03 | 7.644 | 6.17-9.25 |
| Cyclosporin A (µg/L) | 45.387 | 34.06-51.10 | 147.143 | 125.89-188.83 |

**[Table 9-2]**

| | CONTROL | | | |
|---|---|---|---|---|
| | Concentration (in specimen) | | | |
| LEVEL | L3 | | L4 | |
| | Quantitatively assessed value | Reference value | Quantitatively assessed value | Reference value |
| MPA (mg/L) | 42.307 | 34.00-46.00 | | |
| MPAG (mg/L) | 176.374 | 170.00-230.00 | | |
| Everolimus (µg/L) | 13.439 | 9.96-14.94 | 17.602 | 16.58-24.88 |
| Sirolimus (µg/L) | 11.911 | 9.54-14.32 | 19.545 | 16.74-25.12 |
| Tacrolimus (µg/L) | 12.776 | 10.14-15.20 | 18.213 | 14.96-22.44 |
| Cyclosporin A (µg/L) | 833.500 | 622.24-933.36 | 1441.302 | 1089.84-1634.76 |

Referring to the results given in Table 9-1 and Table 9-2, it was demonstrated that the quantitatively-assessed values of all the components of the first immunosuppressant and the second immunosuppressant fit within the reference ranges for all the levels (L1 to L4).

### <<Comparative Experiment: Analysis Results When Second Immunosuppressant Was Ionized in Positive Mode>>

As a comparative experiment, mass spectrometry was carried out in which ionization of the second immunosuppressant (MPA, MPAG) was performed in a positive mode. The analysis conditions were the same as those for creating the calibration curves as described above, except that the ionization mode was changed to a positive mode and the MRM conditions were as described below.

### (MRM Conditions)

MPA (ESI-Positive): 338.20>207.10 (CE -24 V)
MPA IS (ESI-Positive): 342.10>211.30 (CE -14 V)
MPAG (ESI-Positive): 514.10>207.15 (CE -37 V)
MPAG IS (ESI-Positive): 518.10>211.20 (CE -35 V)

Approximate expressions for the calibration curves obtained by the above-described analysis, as well as the proportion of variance, are given below. In each result, it was indicated that ionization in a negative mode yielded a higher proportion of variance. Hence, it is conceivable that for analysis of the second immunosuppressant, ionization needs to be performed in a negative mode.
MPA calibration curve (positive mode)
Approximate expression: Y=0.0786918X+0.0169183
R² (proportion of variance)=0.8985811, R=0.9479352
MPAG calibration curve (positive mode)
Approximate expression: Y=0.00586797X+0.000886828
R² (proportion of variance)=0.9924132, R=0.9961994

The result presented above demonstrated that the blood testing method according to the present invention is capable of quantitatively assessing two or more types of immunosuppressants in the same blood sample.

### [Aspects]

As will be appreciated by those skilled in the art, the above-described example embodiments and examples are specific examples of the below aspects.

(Item 1) A blood testing method according to an aspect is a blood testing method comprising:
a preparation step of preparing whole blood drawn from a subject;
a pretreatment step of pretreating the whole blood to prepare an analysis sample;
a separation step of separating the analysis sample into components by using a liquid chromatograph; and
an analysis step of analyzing the separated components by using a mass spectrometry apparatus, wherein
the subject is a subject receiving administration of a first immunosuppressant and a second immunosuppressant,
the first immunosuppressant includes at least one selected from the group consisting of tacrolimus, cyclosporine A, everolimus, and sirolimus,
the second immunosuppressant includes at least one selected from the group consisting of mycophenolic acid and metabolites thereof,
the separated components include the first immunosuppressant and the second immunosuppressant, and
in the analyzing by using a mass spectrometry apparatus, the analyzing is performed in a positive mode when a component to be analyzed is the first immunosuppressant, and the analyzing is performed in a negative mode when a component to be analyzed is the second immunosuppressant. With the blood testing method according to Item 1, it is possible to quantitatively assess two or more types of immunosuppressants in the same blood sample.

(Item 2) In the blood testing method according to Item 1, the analysis step further includes quantitatively assessing the separated components, based on a calibration curve created in advance. With the blood testing method according to Item 2, it is possible to quantitatively assess the blood concentration of an administered immunosuppressant with more accuracy.

(Item 3) In the blood testing method according to Item 1 or Item 2, the liquid chromatograph is a high performance liquid chromatograph. With the blood testing method according to Item 3, it is possible to carry out quantitative assessment with more accuracy.

(Item 4) In the blood testing method according to any one of Item 1 to Item 3, the mass spectrometry apparatus is a triple quadrupole mass spectrometry apparatus. With the blood testing method according to Item 4, it is possible to simultaneously detect many components in whole blood.

Although the embodiment and examples of the present invention are described above, it is originally planned that certain configurations of the embodiment and examples can be combined as appropriate.

The embodiment and examples disclosed herein are illustrative and nonrestrictive in any respect. The scope of the present invention is defined by the terms of the claims, not by the embodiment and examples, and intended to encompass all modifications and variations equivalent in meaning and scope to the claims.

## Claims

1. A blood testing method comprising:
a preparation step of preparing whole blood drawn from a subject;
a pretreatment step of pretreating the whole blood to prepare an analysis sample;
a separation step of separating the analysis sample into components by using a liquid chromatograph; and
an analysis step of analyzing the separated components by using a mass spectrometry apparatus, wherein
the subject is a subject receiving administration of a first immunosuppressant and a second immunosuppressant,
the first immunosuppressant includes at least one selected from the group consisting of tacrolimus, cyclosporine A, everolimus, and sirolimus,
the second immunosuppressant includes at least one selected from the group consisting of mycophenolic acid and metabolites thereof,
the separated components include the first immunosuppressant and the second immunosuppressant, and
in the analyzing by using a mass spectrometry apparatus, the analyzing is performed in a positive mode when a component to be analyzed is the first immunosuppressant, and the analyzing is performed in a negative mode when a component to be analyzed is the second immunosuppressant.

2. The blood testing method according to claim 1, wherein the analysis step further includes quantitatively assessing the separated components based on a calibration curve created in advance.

3. The blood testing method according to claim 1 or 2, wherein the liquid chromatograph is a high performance liquid chromatograph.

4. The blood testing method according to claim 1 or 2, wherein the mass spectrometry apparatus is a triple quadrupole mass spectrometry apparatus.
